# FASCICULE DE BREVET EUROPEEN

(11) **EP 2 159 309 B1**
(45) Date de publication et mention de la délivrance du brevet: **08.06.2011**
(21) Numéro de dépôt: 09168453.0
(22) Date de dépôt: 24.08.2009
(51) Int. Cl.: D01F 9/14, C07C 253/00

(54) **Procédé de fabrication de fibres de carbone**
Verfahren zur Herstellung von Karbonfasern
Method for manufacturing carbon fibres

(30) Priorité: 25.08.2008 FR 0855703
(43) Date de publication de la demande: 03.03.2010
(73) Titulaire: ARKEMA FRANCE, 92700 Colombes (FR)
(72) Inventeur: Plee, Dominique, 64140, LONS (FR)

(56) Documents cités:
- DE-A1- 3 037 582
- FR-A- 2 882 052
- FR-A- 2 884 818
- US-A1- 2007 196 648

## Description

La présente invention concerne un procédé de fabrication de fibres de carbone à partir de matières premières d'origine renouvelable, ainsi que les fibres susceptibles d'être obtenues selon ce procédé.

Les fibres de carbone sont des matériaux constitués de fibres très fines de 5 à 10 microns de diamètre dont le carbone est l'élément chimique principal. D'autres atomes sont généralement présents tels que l'oxygène, l'azote, l'hydrogène et moins souvent le soufre. Les atomes de carbone sont liés entre eux et forment des cristaux de type graphitique plus ou moins parallèles à l'axe de la fibre. Plusieurs milliers de ces fibres sont torsadées ensemble pour former un toron. Ces torons peuvent être utilisés seuls ou mis sous la forme de tissu.

Les fibres de carbone sont un des matériaux dont le développement est le plus rapide à l'heure actuelle. Utilisées comme renforts de matériaux thermodurcissables tels que les résines époxy et les résines polyesters réticulées, ou comme agents d'amélioration de certains thermoplastiques tels que les polyamides, elles permettent en effet d'obtenir des composites très résistants par rapport à leur poids et même souvent plus résistants que l'acier par unité de poids.

Ces composites présentent ainsi de très bons modules de traction, une très grande résistance à la rupture et un coefficient de dilatation thermique faible. Ils peuvent remplacer les métaux dans beaucoup d'applications, par exemple des pièces d'avions ou d'engins spatiaux, dans les équipements de sport (raquettes de tennis et clubs de golf) et dans les résines structurales employées dans les éoliennes. Les fibres de carbone trouvent également des applications dans la filtration de gaz à haute température, comme agents antistatiques, comme électrodes de spécialité du fait de leur résistance à la corrosion, en tant que renfort des réservoirs de gaz sous pression, notamment pour le stockage d'hydrogène.

L'origine des fibres de carbone remonte à 1958, date à laquelle elles furent proposées par Union Carbide. Les premières fibres étaient de qualité médiocre mais des améliorations rapides furent obtenues en utilisant le polyacrylonitrile (PAN) comme précurseur. Selon le précurseur, les fibres ne sont pas identiques : celles obtenues à partir de PAN sont plutôt turbostratiques, avec des plans graphitiques qui présentent un angle non nul entre eux et qui sont plissés de manière aléatoire. Les fibres obtenues à partir de brai sont graphitiques après traitement thermique à plus de 2200°C. Les fibres obtenues à partir de ces différents précurseurs ont donc également des propriétés différentes. Ainsi, celles obtenues à partir de PAN présentent en général une meilleure résistance à la rupture.

La synthèse de fibres de carbone à partir de PAN comprend typiquement une étape de cuisson de la fibre à 300°C environ, afin de l'oxyder légèrement, après quoi la fibre est placée dans une atmosphère inerte sous argon ou azote à une température de l'ordre de 2000°C dans un four électrique. Après cette étape, la fibre contient de l'ordre de 95% de carbone. Un traitement ultérieur à plus haute température augmente sa conductivité thermique et son module.

On comprend donc que les fibres de carbone sont des produits très coûteux en énergie, du fait des hautes températures nécessaires lors de leur fabrication. Cette consommation d'énergie n'est pas compensée par leur aptitude à permettre la fabrication de véhicules plus légers et à diminuer en conséquence la consommation énergétique dans les applications de transport.

Dans les pays où la production électrique provient majoritairement de la consommation de ressources fossiles, cette dépense énergétique s'accompagne d'une augmentation de l'émission des gaz à effet de serre, particulièrement dommageable pour l'environnement. Pour réduire cette émission, il n'est pas facile d'agir sur le procédé de synthèse des fibres de carbone. En revanche, il semble possible d'employer des matières premières à plus faible impact environnemental que le PAN classiquement utilisé dans la synthèse des fibres de carbone, en particulier des matières premières renouvelables ou bioressourcées.

Dans ce contexte, la présente invention a pour objet un nouveau procédé de synthèse de fibres de carbone à partir de matières premières d'origine renouvelable ou bioressourcées.

Plus précisément, l'invention a pour objet un procédé de fabrication de fibres de carbone, comprenant :
a) la synthèse d'acroléine à partir de glycérol d'origine végétale,
b) l'ammoxidation de l'acroléine pour obtenir de l'acrylonitrile,
c) la polymérisation de l'acrylonitrile en homo- ou copolymère d'acrylonitrile (PAN),
d) la transformation du PAN en fibres de PAN,
e) l'oxydation partielle des fibres de PAN, et
f) la carbonisation des fibres de PAN partiellement oxydées.

Ce procédé sera maintenant décrit plus en détail. Il convient de noter, à titre liminaire, qu'il peut comprendre d'autres étapes que celles mentionnées ci-dessus et en particulier une ou plusieurs étapes préliminaires à l'étape (a), une ou plusieurs étapes postérieures à l'étape (f) et/ou une ou plusieurs étapes intermédiaires, pour autant que ces étapes n'affectent pas négativement l'ensemble du procédé, en particulier le rendement et/ou la qualité des fibres de carbone obtenues.

Dans la première étape du procédé selon l'invention, de l'acroléine est formée à partir de glycérol d'origine végétale.

Selon une forme d'exécution préférée de l'invention, le glycérol utilisé dans l'étape (a) est obtenu comme sous-produit d'une transestérification de triglycérides d'origine végétale. Une réaction classique de transestérification met en effet en oeuvre un mono-alcool linéaire en C₁-C₁₀, tel que le méthanol ou l'éthanol, ou un mono-alcool cyclique en C₃-C₆, qui est mis à réagir sur des triglycérides pour obtenir des alkylesters d'alcools en C₁-C₁₀ et du glycérol. Les triglycérides sont des composés de formule : R₁-CO-O-CH₂-CH(OCO-R₂)-CH₂-O-CO-R₃, dans lesquels R₁ à R₃ désignent des groupes alkyle linéaire ou ramifiés, saturés ou (poly) insaturés, en C₁₀-C₃₀, par exemple en C₁₂-C₁₈, qui sont un constituant important des huiles et graisses végétales telles que l'huile de palme, l'huile de graines de lin, l'huile d'arachide, l'huile de coco, l'huile de tournesol, l'huile de soja ou l'huile de colza. Cette dernière est notamment transestérifiée dans la fabrication du biodiesel préconisé comme carburant en substitution des combustibles fossiles. Le glycérol utilisé selon l'invention peut donc être un sous-produit dans la fabrication de biodiesel à partir d'huile végétale. A titre indicatif, environ 1 tonne de glycérol est obtenue à partir de 10 tonnes de triglycérides d'acides gras.

L'étape de transestérification est généralement conduite à une température de 20 à 150°C, de préférence de 25 à 100°C, plus préférentiellement de 25 à 80°C, par exemple pendant une durée de 4 à 8 heures, en présence de catalyseurs acides ou basiques, de préférence en présence d'un catalyseur basique tel que le méthoxyde de sodium ou de potassium, dans un solvant tel que le méthanol, dans un réacteur agité (en particulier sous fort cisaillement) ou à lit fixe ou fluidisé. En variante, la transestérification peut être réalisée en présence de méthanol supercritique à haute température et pression. En général, tous les réactifs sont déshydratés pour éviter la saponification des triglycérides et permettre une séparation plus aisée du glycérol.

Dans l'étape (a) du procédé selon l'invention, le glycérol est déshydraté en acroléine en phase liquide ou en phase gazeuse. Selon une forme d'exécution préférée de l'invention, la déshydratation du glycérol en acroléine est effectuée à 250-350°C et 1 à 5 bars, en présence d'oxygène moléculaire et avantageusement d'un catalyseur solide acide, comme décrit dans la demande WO 2006/087083, de préférence en présence d'un catalyseur de type acide fort ayant un indice d'acidité de Hammett Ho compris entre -9 et -18, comme décrit dans la demande WO 2006/087084.

En variante, la déshydratation du glycérol peut être réalisée comme décrit dans la demande US 2008/119663, par chauffage à une température allant de 250 à 400°C, de préférence de 260 à 300°C. Dans le cas d'une réaction en phase liquide, la pression est ajustée, par exemple entre 1 et 50 bars, de manière à maintenir le milieu réactionnel à l'état liquide. Un catalyseur acide homogène ou hétérogène, et/ou des sels d'acides minéraux, tels que les (hydrogéno)sulfates de potassium ou de sodium, peuvent être utilisés pour accélérer la réaction. On peut ainsi avoir recours à un catalyseur acide homogène tel que l'acide sulfurique, l'acide phosphorique, l'acide toluène sulfonique ou l'acide méthanesulfonique ou à un catalyseur hétérogène tel qu'une zéolite du type HZSM-5 ou MCM-22, des oxydes métalliques, tels que l'oxyde d'aluminium, recouverts d'un acide inorganique tel que l'acide phosphorique, ou une résine échangeuse d'ions. En variante, il est possible d'utiliser des biocatalyseurs tels que des lipases ou des estérases.

La présente description n'exclut pas que le glycérol puisse être déshydraté en acroléine simultanément à sa formation à partir des triglycérides, en présence d'un catalyseur de déshydratation tel que décrit ci-dessus, comme enseigné dans le document US 2008/0119663. Il conviendra dans ce cas de procéder ensuite à une séparation de l'acroléine du milieu réactionnel par tout moyen approprié, par exemple par distillation, extraction, séparation de phase ou à l'aide de membranes. On préfère toutefois que les étapes de transestérification et de déshydratation soient conduites séparément, afin d'optimiser le rendement en acroléine. Pour ce faire, le glycérol est avantageusement extrait du milieu réactionnel de transestérification par distillation, séparation par membrane ou séparation de phase. Il peut éventuellement être ensuite purifié pour éliminer les savons, sels et bases qu'il contient en faible quantité, avant d'être transformé en acroléine.

L'acroléine produite dans l'étape (a) du procédé selon l'invention est ensuite soumise, dans l'étape (b), à une ammoxidation pour obtenir de l'acrylonitrile, selon le schéma réactionnel suivant :

CH₂=CH-CHO + NH₃ + 0,5 O₂ → CH₂=CH-CN + 2 H₂O

Cette étape d'ammoxidation est bien connue de l'homme du métier et peut notamment être conduite à 200-450°C en faisant passer un mélange d'acroléine, d'ammoniac, d'air et de gaz inerte sur un catalyseur constitué d'un ou plusieurs sels oxygénés d'arsenic et d'éléments moins électronégatifs. Un procédé de ce type est décrit dans la demande FR 1 410 967. En variante, elle peut être réalisée comme décrit dans le document DE-1 070 170, en utilisant un catalyseur à base de molybdène, à une température de 250-350°C, ou comme décrit dans le document US-3,094,552, en faisant passer le mélange réactionnel sur un catalyseur à base d'étain et d'antimoine, à une température de 300-550°C, ou encore comme décrit dans le brevet GB-709 337, en présence d'un catalyseur constitué d'un mélange de silice, d'oxyde de molybdène et d'acide phosphorique, à une température de 250-600°C.

L'acrylonitrile ainsi obtenu est polymérisé, dans l'étape (c) du procédé selon l'invention. L'acrylonitrile peut être homopolymérisé ou, selon une forme d'exécution préférée de l'invention, copolymérisé avec au moins un autre monomère, de préférence un monomère acrylique, c'est-à-dire d'un monomère d'acide (méth)acrylique ou d'alkylester d'acide (méth)acrylique, tel que l'acrylate de méthyle, le méthacrylate de méthyle ou l'acide acrylique. En effet, pour autant qu'il ne représente pas plus de 10% en poids, par rapport au poids total des monomères à polymériser, ce co-monomère permet un meilleur contrôle des effets thermiques dans l'étape (e) ultérieure (Gupta, A.K. et al., JMS-Rev. Macromol. Chem. Phys. C31, 1991). Ce co-monomère peut lui-même être d'origine renouvelable. Ainsi, l'acide acrylique peut être obtenu à partir de glycérol et le méthacrylate de méthyle peut incorporer par sa synthèse de l'acétone et du méthanol d'origine renouvelable. L'acrylate de méthyle est préféré pour une utilisation dans la présente invention, car il est très proche en polarité de l'acrylonitrile.

La (co)polymérisation de l'acrylonitrile peut être effectuée de manière classique, par polymérisation radicalaire en solution, en utilisant un solvant de type diméthylsulfoxyde (DMSO) ou diméthylformamide (DMF), éventuellement en présence d'un activateur tel que l'azobisisobutyronitrile (AIBN) ou d'un ester d'acide azocarboxylique, comme décrit dans la demande de brevet US 2004/068069. En variante, la (co)polymérisation de l'acrylonitrile peut être effectuée en dispersion aqueuse en présence de thiocyanate de sodium, de chlorure de zinc ou de perchlorate de sodium, par exemple. Des procédés de polymérisation utilisables sont notamment décrits dans "Polymerization of acrylic fibers", Encyclopedia of Polymer Science, Vol. 1, pp. 334-338, 1985. L'homo- ou copolymère obtenu, désigné par PAN, a en général une masse moléculaire moyenne en poids de l'ordre de 80.000 à 120.000 g/mol.

Ce PAN est ensuite mis sous forme de fibres dans l'étape (d) du procédé selon l'invention. Cette étape peut être mise en oeuvre de plusieurs façons.

Selon une méthode, on mélange le PAN avec au moins un plastifiant tel qu'un carbonate d'alkyle, en particulier le carbonate d'éthylène (ester d'éthylène glycol et d'acide carbonique), on le chauffe à 110-160°C pour le ramollir et on l'injecte à travers une filière fine, de manière à ce qu'il retombe dans un bain, constitué par exemple d'eau, où il coagule et se solidifie sous forme de fibres. Ce procédé est tout à fait semblable à celui que l'on utilise pour fabriquer des fibres acryliques textiles.

Selon une autre méthode, on dissout le PAN dans un solvant (DMSO, DMF, DMA ou solution aqueuse de sels inorganiques) et on l'injecte par une filière fine dans une chambre de réception ou four de séchage où, après évaporation du solvant, le polymère forme une fibre solide.

Dans tous les cas, les fibres ainsi obtenues sont lavées et étirées jusqu'à obtenir le diamètre de fibre voulu. L'étirage permet en outre d'aligner les espèces moléculaires, ce qui facilitera par la suite, lors de la carbonisation, une formation correcte des liaisons carbone-carbone et assurera à la fibre une grande solidité.

Avant la carbonisation proprement dite, les fibres ont besoin d'être légèrement modifiées chimiquement afin de convertir leur arrangement atomique vers une structure plus réticulée. Cette opération dite de stabilisation ou d'oxydation partielle constitue l'étape (e) du procédé selon l'invention.

Cette opération est réalisée en chauffant à 200-300°C pendant quelques dizaines de minutes les fibres de PAN en présence d'air. De cette manière, la fibre modifie son arrangement atomique et des fonctions de surface polaires sont créées ; elle passe d'un état plastique à un état infusible thermiquement stable. Cette réaction étant exothermique, il convient de veiller à contrôler les transferts thermiques car un emballement pourrait se produire.

Après l'opération de stabilisation, on effectue la carbonisation proprement dite, qui constitue l'étape (f) du procédé selon l'invention, en chauffant les fibres issues de l'étape (e) à une température comprise entre 1200 et 1500°C dans un four balayé d'un gaz inerte et maintenu à une pression supérieure à la pression atmosphérique, afin d'empêcher l'air de rentrer dans le four.

Pendant la carbonisation, la plupart des atomes, à l'exception du carbone, sont expulsés sous forme de vapeur d'eau pour l'oxygène et l'hydrogène, d'ammoniac et de cyanure d'hydrogène pour les atomes d'azote, d'azote gazeux, de monoxyde et dioxyde de carbone provenant des fonctions polaires de surface. L'expulsion de ces atomes permet au carbone de s'organiser sous forme microcristalline en créant des liaisons fortes. La carbonisation peut éventuellement être effectuée en deux étapes, à deux températures différentes, pour un meilleur contrôle de l'ensemble du procédé. La première étape de carbonisation peut ainsi être effectuée à 400-800°C, la fibre étant éventuellement allongée durant cette étape.

A l'issue de cette étape de carbonisation, on obtient des fibres dites « haute résistance « ou « module intermédiaire » selon la température de traitement. Une étape ultérieure de graphitisation entre 2000 et 3000°C permet éventuellement d'obtenir des fibres dites « haut module »

Après la carbonisation/graphitisation, d'autres étapes peuvent être mises en oeuvre, en vue d'améliorer le contact de la fibre avec la matrice dans laquelle elle sera incorporée. On peut ainsi oxyder légèrement sa surface, soit par traitement en présence d'air ou de dioxyde de carbone, soit par traitement en phase liquide avec l'hypochlorite de sodium, l'acide nitrique ou une solution d'acide sulfurique, de soude et de bicarbonate d'ammonium, par exemple. Toutes ces opérations doivent être bien contrôlées pour éviter la création de défauts de surface qui provoqueraient des adhésions défectueuses sur les matrices.

Les fibres peuvent également être soumises à un traitement d'ensimage visant à les protéger pendant leur transport, leur tissage ou leur enroulement. Ce traitement consiste à appliquer sur les fibres un matériau de revêtement choisi pour être compatible avec les agents d'adhésion utilisés dans la fabrication des composites et qui peut par exemple être sélectionné parmi les résines époxydes, les polyesters ou les polyuréthanes.

La présente invention a également pour objet les fibres de carbone susceptibles d'être obtenues suivant le procédé décrit précédemment.

Ces fibres de carbone se caractérisent en ce qu'elles comprennent une quantité non négligeable de carbone d'origine renouvelable ou d'origine bio ou encore d'origine contemporaine, c'est-à-dire de ¹⁴C. En effet, tous les échantillons de carbone tirés d'organismes vivants, et en particulier de la matière végétale utilisée dans la première étape du procédé selon l'invention, sont un mélange de trois isotopes : ¹²C, ¹³C et ¹⁴C dans un rapport ¹⁴C/¹²C maintenu constant par échange continu du carbone avec l'environnement et qui est égal à 1,2 x 10⁻¹². Bien que le ¹⁴C soit radioactif et que sa concentration décroisse donc au cours du temps, sa demi-vie est de 5730 ans, de sorte qu'on estime que la teneur en ¹⁴C est constante depuis l'extraction de la matière végétale jusqu'à la fabrication des fibres et même jusqu'à la fin de leur utilisation.

Plus précisément, on considère que les fibres de carbone selon l'invention ont un rapport isotopique de leur teneur en ¹⁴C à leur teneur en ¹²C qui est supérieur à 10⁻¹², où C¹⁴ représente l'isotope à 6 protons et 8 neutrons alors que C¹² représente l'isotope stable à 6 protons et 6 neutrons mais qui est au plus égale à 1,2 x 10⁻¹². Une fibre de carbone contenant 100% de carbone d'origine renouvelable contient au plus 1,2 x 10⁻¹² de ¹⁴C.

La teneur en ¹⁴C des fibres de carbone peut être mesurée selon des techniques bien connues de datation des restes archéologiques, des bois anciens, des os, de la tourbe ou même des coquillages. Elle peut par exemple être mesurée selon les techniques suivantes :
- Par spectrométrie à scintillation liquide : cette méthode consiste à compter des particules "bêta" issues de la désintégration du ¹⁴C. On mesure le rayonnement bêta issu d'un échantillon de masse connue (nombre d'atomes de carbone connu) pendant un certain temps. Cette "radioactivité" est proportionnelle au nombre d'atomes de ¹⁴C, que l'on peut ainsi déterminer. Le ¹⁴C présent dans l'échantillon émet des rayonnements bêta, qui au contact du liquide scintillant (scintillateur) donnent naissance à des photons. Ces photons ont des énergies différentes (comprises entre 0 et 156 Kev) et forment ce que l'on appelle un spectre de ¹⁴C. Selon deux variantes de cette méthode, l'analyse porte soit sur le CO₂ préalablement produit par l'échantillon carboné dans une solution absorbante appropriée, soit sur le benzène après conversion préalable de l'échantillon carboné en benzène.
- Par spectrométrie de masse : l'échantillon est réduit en graphite ou en CO₂ gazeux, puis analysé dans un spectromètre de masse. Cette technique utilise un accélérateur et un spectromètre de masse pour séparer les ions ¹⁴C des ¹²C et donc déterminer le rapport des deux isotopes.

Ces méthodes de mesure de la teneur en ¹⁴C des matériaux sont décrites précisément dans les normes ASTM D 6866 (notamment D6866-06) et dans les normes ASTMD 7026 (notamment 7026-04). Ces méthodes mesurent le rapport ¹⁴C/¹²C d'un échantillon et le comparent avec le rapport ¹⁴C/¹²C d'un échantillon référence d'origine 100% renouvelable, pour donner un pourcentage relatif de carbone d'origine renouvelable dans l'échantillon.

La présente invention a donc également pour objet des fibres de carbone présentant un rapport isotopique de leur teneur en ¹⁴C à leur teneur en ¹²C qui est supérieur à 2 × 10⁻¹¹, par exemple supérieur à 5 x 10⁻¹¹ et mieux à 10⁻¹², et au plus égal à 1,2 x 10⁻¹².

Les fibres de carbone selon l'invention peuvent être utilisées dans toutes les applications où elles sont habituellement mises en oeuvre, notamment pour le renfort des matériaux composites, en particulier dans la fabrication de pièces d'avions ou d'engins spatiaux, d'équipements de sport (raquettes de tennis et clubs de golf) et d'éoliennes ; dans la filtration de gaz à haute température ; comme agents antistatiques ; comme électrodes de spécialité ; ou comme renfort des réservoirs de gaz sous pression, notamment pour le stockage d'hydrogène.

La présente invention a donc également pour objet ces utilisations des fibres de carbone décrites précédemment.

## Revendications

1. Procédé de fabrication de fibres de carbone, comprenant :
a) la synthèse d'acroléine à partir de glycérol d'origine végétale,
b) l'ammoxidation de l'acroléine pour obtenir de l'acrylonitrile,
c) la polymérisation de l'acrylonitrile en homo- ou copolymère d'acrylonitrile (PAN),
d) la transformation du PAN en fibres de PAN,
e) l'oxydation partielle des fibres de PAN, et
f) la carbonisation des fibres de PAN partiellement oxydées.

2. Procédé selon la revendication 1, **caractérisé en ce que** le glycérol utilisé dans l'étape (a) est obtenu comme sous-produit d'une transestérification de triglycérides d'origine végétale.

3. Procédé selon la revendication 2, **caractérisé en ce que** le glycérol est obtenu comme sous-produit dans la fabrication de biodiesel à partir d'huile végétale.

4. Procédé selon l'une quelconque des revendications 1 à 3, **caractérisé en ce que** le glycérol est déshydraté en acroléine, dans l'étape (a), par chauffage à une température allant de 250 à 400°C, de préférence de 260 à 300°C.

5. Procédé selon l'une quelconque des revendications 1 à 4, **caractérisé en ce que** l'acrylonitrile est copolymérisé, dans l'étape (c), avec au moins un co-monomère acrylique tel que l'acrylate de méthyle, le méthacrylate de méthyle ou l'acide acrylique.

6. Procédé selon la revendication 5, **caractérisé en ce que** le co-monomère ne représente pas plus de 10% en poids, par rapport au poids total des monomères à polymériser.

7. Procédé selon l'une quelconque des revendications 1 à 6, **caractérisé en ce que**, dans l'étape (e), l'oxydation partielle des fibres de PAN est réalisée en chauffant les fibres à 200-300°C pendant quelques minutes en présence d'air.

8. Procédé selon l'une quelconque des revendications 1 à 7, **caractérisé en ce que,** dans l'étape (f), la carbonisation des fibres de PAN est effectuée en chauffant les fibres à une température comprise entre 1200 et 1500°C dans un four balayé d'un gaz inerte et maintenu à une pression supérieure à la pression atmosphérique.

9. Fibres de carbone susceptibles d'être obtenues suivant le procédé selon l'une quelconque des revendications 1 à 8.

10. Fibres de carbone présentant un rapport isotopique de leur teneur en ¹⁴C à leur teneur en ¹²C qui est supérieur à 5 x 10⁻¹¹ et mieux à 10⁻¹².

11. Utilisation des fibres de carbone selon la revendication 9 ou 10 pour le renfort des matériaux composites, en particulier dans la fabrication de pièces d'avions ou d'engins spatiaux, d'équipements de sport (raquettes de tennis et clubs de golf) et d'éoliennes ; dans la filtration de gaz à haute température ; comme agents antistatiques ; comme électrodes de spécialité ; ou comme renfort des réservoirs de gaz sous pression, notamment pour le stockage d'hydrogène.

## Claims

1. Method of manufacturing carbon fibres, comprising;
a) synthesis of acrolein from glycérol of vegetable origin;
b) ammoxidation of the acrolein to obtain acrylonitrile;
c) polymerization of the acrylonitrile to a homopolymer or copolymer of acrylonitrile (PAN) ;
d) conversion of the PAN to PAN fibres;
e) partial oxidation of the PAN fibres; and
f) carbonization of the partially oxidized PAN fibres.

2. Method according to Claim 1, **characterized in that** the glycerol used in step (a) is obtained as a by-product of a transesterification of triglycérides of vegetable origin.

3. Method according to Claim 2, **characterized in that** the glycerol is obtained as a by-product in the manufacture of biodiesel from vegetable oil.

4. Method according to any one of Claims 1 to 3, **characterized in that** the glycerol is dehydrated to acrolein, in step (a), by heating at a temperature ranging from 250 to 400°C, preferably from 260 to 300°C.

5. Method according to any one of Claims 1 to 4, **characterized in that** the acrylonitrile is copolymerized, in step (c), with at least one acrylic comonomer such as methyl acrylate, methyl methacrylate or acrylic acid.

6. Method according to Claim 5, **characterized in that** the comonomer does not represent more than 10% by weight relative to the total weight of the monomers to be polymerized.

7. Method according to any one of Claims 1 to 6, **characterized in that**, in step (e), the partial oxidation of the PAN fibres is carried out by heating the fibres at 200-300°C for a few minutes in the presence of air.

8. Method according to any one of Claims 1 to 7, **characterized in that**, in step (f), the carbonization of the PAN fibres is carried out by heating the fibres at a temperature between 1200 and 1500°C in a furnace purged with an inert gas and maintained at a pressure above atmospheric pressure.

9. Carbon fibres capable on being obtained following the method according to any one of Claims 1. to 8.

10. Carbon fibres having an isotopic ratio of their ¹⁴C content to their ¹²C content which is greater than 5 × 10⁻¹¹ and preferabily greater than 10^{-12.}

11. Use of the carbon fibres according to Claim 9 or 10 for reinforcing composites, in particular in the manufacture of aircraft or spacecraft parts, sports equipment (tennis rackets and golf clubs) and wind turbines; in the filtration of high-temperature gases; as antistatic agents; as speciality electrodes; or as reinforcement for pressurized gas tanks, especially for storing hydrogen.

## Patentansprüche

1. Verfahren zur Herstellung von Kohlenstofffasern, das die folgenden Schritte umfasst:
a) Synthese von Acrolein aus Glycerol pflanzlicher Herkunft;
b) Ammoxidation des Acroleins zur Herstellung von Acrylnitril;
c) Polymerisation von Acrylnitril als Homo- oder Copolymer von Acrylnitril (PAN);
d) Umformung des PAN in PAN-Fasern;
e) partielle Oxidation der PAN-Fasern.;
f) Carbonisierung der partiell oxidierten PAN-Fasern.

2. Verfahren nach Anspruch 1, **dadurch gekennzeichnet, dass** das in Schritt (a) eingesetzte Glycerol als Nebenprodukt bei der Umesterung von Triglyceriden pflanzlicher Herkunft erhalten wird.

3. Verfahren nach Anspruch 2, **dadurch gekennzeichnet, dass** das eingesetzte Glycerol als Nebenprodukt bei der Herstellung von Biodiesel aus pflanzlichem Öl erhalten wird.

4. Verfahren nach einem der Ansprüche 1. bis 3, **dadurch gekennzeichnet, dass** das Glycerol in Schritt (a) durch Erwärmen auf eine Temperatur im Bereich von 250 bis 400 °C und vorzugsweise 260 bis 300 °C zu Acrolein dehydratisiert wird.

5. Verfahren, nach einem der Ansprüche 1 bis 4, **dadurch gekennzeichnet, dass** in Schritt (c) das Acrylnitril mit mindestens einem Acrylcomonomer copolymerisiert wird, wie Methylacrylat, Methylmethacrylat oder Acrylsäure.

6. Verfahren nach Anspruch 5, **dadurch gekennzeichnet, dass** das Comonomer nicht mehr als 10 Gew.-%, bezogen auf das Gesamtgewicht der zu polymerisierenden Monomere, ausmacht.

7. Verfahren nach einem der Ansprüche 1 bis 6, **dadurch gekennzeichnet, dass** in Schritt (e) die partielle Oxidation der PAN-Fasern realisiert wird, indem die Fasern in Gegenwart von Luft einige Minuten auf 200 300 °C erwärmt werden.

8. Verfahren nach einem der Ansprüche 1 bis 7, **dadurch gekennzeichnet**, dans in Schritt (f) die Carbonisierung der PAN-Fasern durchgeführt wird, indem die Fasern in einem mit Inertgas gespülten und auf einem Druck über Atmosphärendruck gehaltenen Offen auf eine Temperatur im Bereich von 1200 bis 1500 °C erwärmt werden.

9. Kohlenstofffasern, die gemäß dem Verfahren nach einem der Ansprüche 1 bis 8 erhältlich, sind.

10. Kohlenstofffasern, die ein Isotopenverhältnis ihres Gehalt an ¹⁴C und ihres Gehalts an ¹²C über 5 x 10⁻¹¹ und besser 10⁻¹² aufweisen.

11. Verwendung von Kohlenstofffasern nach Anspruch 9 oder 10 zur Verstärkung von Verbundwerkstoffen, insbesondere bei der Herstellung von Flugzeugteilen oder Teilen von Raumfahrzeugen, Sportausrüstungen (Tennisschlägern und Golfschlägern) und Windkraftanlagen; für die Gasfiltration bei hoher Temperatur; als Antistatika; als Spezialelektroden; oder als Verstärkung von Gasbehältern unter Druck, insbesondere zur Aufbewahrung von Wasserstoff.
